(19) Europäisches Patentamt
European Patent Office
Office européen des brevets

(11) Veröffentlichungsnummer: **0 167 995**
**B1**

# (12) EUROPÄISCHE PATENTSCHRIFT

(45) Veröffentlichungstag der Patentschrift: **04.01.89**

(51) Int. Cl.⁴: **C 07 D 403/10,** C 07 D 403/04, A 61 K 31/50

(21) Anmeldenummer: **85108319.6**

(22) Anmeldetag: **05.07.85**

(54) **(Pyrrol-1-yl)-phenyl-dihydropyridazinone, ihre Herstellung und Verwendung.**

(30) Priorität: **12.07.84 DE 3425632**

(43) Veröffentlichungstag der Anmeldung:
**15.01.86 Patentblatt 86/3**

(45) Bekanntmachung des Hinweises auf die Patenterteilung:
**04.01.89 Patentblatt 89/1**

(84) Benannte Vertragsstaaten:
**AT BE CH DE FR GB IT LI NL SE**

(56) Entgegenhaltungen:
**EP-A-0 075 436**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Geiss, Karl- Heinz, Dr., Kirchenstrasse 8, D-6711 Beindersheim (DE)**
Erfinder: **Schmied, Bernhard, Dr., Ringstrasse 1, D-6901 Dossenheim (DE)**
Erfinder: **Raschack, Manfred, Dr., Donnersbergstrasse 7, D-6714 Weisenheim am Sand (DE)**
Erfinder: **Lehmann, Hans- Dieter, Prof. Dr., Im Hefen 15, D-6945 Hirschberg (DE)**
Erfinder: **Gries, Josef, Dr., Roemerweg 43, D-6706 Wachenheim (DE)**
Erfinder: **Ruebsamen, Klaus, Dr., Erschigweg 19, 6730 Neustadt (DE)**

**EP 0 167 995 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue (Pyrrol-1-yl)-phenyl-dihydropyridazinone, Verfahren zu deren Herstellung sowie deren Verwendung bei der Bekämpfung von Krankheiten.

6-Aryldihydropyridazinone mit pharmakologischen Wirkungen sind bereits bekannt (vgl. J. Med. Chem. 17, 273 (1974), J. Het. Chem. 11, 755 (1974), J. Med. Chem. 26, 800 (1983)). Eine Reihe von 6-phenyl-dihydropyridazinonen, die die myokardiale Kontraktilität erhöhen und antihypertensiv wirken, sind in der EP-A-75 436 beschrieben.

Es wurde nun gefunden, daß (Pyrrol-1-yl)-phenyl-dihydropyridazinone der Formel I

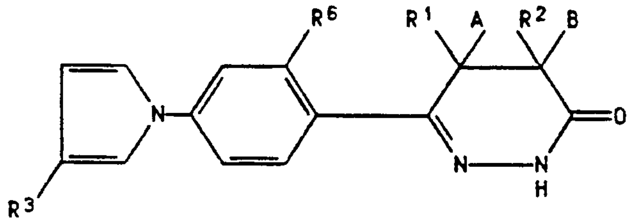 I,

worin

$R^1$ ein Wasserstoffatom, eine Methylgruppe oder eine Hydroxymethylgruppe,

$R^2$ ein Wasserstoffatom oder eine Methylgruppe, oder $R^1$ und $R^2$ zusammen einen Methylen- oder Ethylenrest,

A und B Wasserstoffatome oder - falls $R^1$ und $R^2$ keine Alkylengruppe darstellen - auch eine gemeinsame Bindung sind,

$R^3$ eine Formyl- oder Hydroxymethylgruppe oder eine Gruppe $CH_2$-$NR^4R^5$ bedeutet, worin $R^4$ eine $C_{1-6}$-Alkylgruppe oder eine Phenyl-$C_{1-3}$-alkylengruppe und $R^5$ ein Wasserstoffatom oder eine $C_{1-6}$-Alkylgruppe oder die Formylgruppe sind, oder $R^4$ und $R^5$ zusammen einen $C_{4-7}$-Alkylenrest bedeuten, und

$R^6$ ein Wasserstoffatom bedeutet oder zusammen mit $R^1$ den Rest -$(CH_2)_m$- bedeutet, worin m für 0, 1, 2 oder 3 steht.

sowie gegebenenfalls deren Säureadditionssalze wertvolle pharmakologische Eigenschaften besitzen.

Die Verbindungen der Formel I, in denen $R^3$ die Gruppe $CH_2$-$NR^4R^5$ und $R^5$ keine Formylgruppe bedeutet, können mit Sauren Salze bilden. Als Sauren kommen solche in Frage, die physiologisch vertraglich sind. Hierzu gehören insbesondere Schwefelsäure, Phosphorsäure, Amidosulfonsäure. Salpetersäure oder organische Säuren, wie Essigsäure, Propionsäure, Oxalsäure, Malonsäure, Sernsteinsäure, Fumarsäure, Maleinsäure, Zitronensäure, Weinsäure, Milchsäure.

$R^1$ bedeutet bevorzugt ein Wasserstoffatom oder die Methylgruppe, insbesondere die Methylgruppe.

$R^2$ bedeutet bevorzugt ein Wasserstoffatom, oder $R^1$ und $R^3$ bilden bevorzugt einen Methylen- oder Ethylenrest, insbesondere den Methylenrest,

A und B bedeuten bevorzugt Wasserstoffatome und

$R^6$ bedeutet bevorzugt ein Wasserstoffatom oder bildet zusammen mit $R^1$ den Rest -$(CH_2)_m$-, wobei m für 1 oder 2 steht, insbesondere bedeutet $R^6$ ein Wasserstoffatom.

Die neuen Verbindungen werden hergestellt, indem man p-Aminophenyl-dihydropyridazinone der Formel II

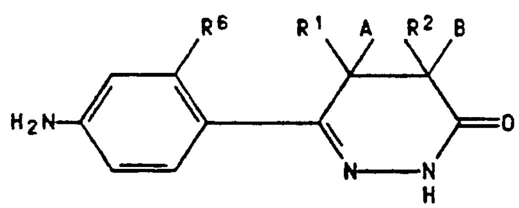 II.

worin $R^1$, $R^2$, $R^6$, A und B die angegebene Bedeutung besitzen mit Tetrahydrofuranen der Formel III

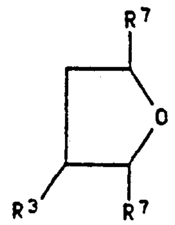

III,

worin $R^3$ die angegebene Bedeutung hat und $R^7$ $C_{1-4}$-Alkoxy, $C_{1-4}$-Acyloxyreste, Chlor- oder Bromatome bedeuten, umsetzt, und die so erhaltenen Verbindungen - falls $R^3$ = Formyl - gegebenenfalls zu den Verbindungen I, $R^3 = CH_2OH$ reduziert oder durch reduktive Aminierung in die Amine I, $R^3 = CH_2NR^4R^5$ überführt.

Bevorzugte Reste für $R^7$ sind $C_{1-4}$-Alkoxyreste, insbesondere Methoxyreste.

Die Herstellung der neuen Verbindungen erfolgt entweder in einer niederen Alkancarbonsäure, wie Essigsäure, oder in einem organischen Lösungsmittel unter Zusatz eines sauren Katalysators, wie einer anorganischen Säure, einer organischen Carbonsäure oder einer Sulfonsäure. Sie kann auch in Gegenwart eines sauer beladenen Ionenaustauschers durchgefuhrt werden. Der Katalysator wird in einer Menge von 0,01 bis 300 Molprozent eingesetzt, wobei zur Darstellung der Verbindungen I mit $R^3$ = Hydroxymethyl oder Formyl oder $CH_2$-$NR^4R^5$, falls $R^5$ eine Formylgruppe bedeutet, 0,01 bis 20, vorzugsweise 0,1 bis 10 Molprozent und zur Darstellung der Verbindungen I mit $R^3 = CH_2$-$NR^4R^5$, worin $R^5$ keine Formylgruppe bedeutet, 100 bis 300, vorzugsweise 100 bis 150 Molprozent eingesetzt werden. Die Umsetzung kann bei Normaldruck oder erhöhtem Druck zwischen Raumtemperatur und der Rückflußtemperatur des eingesetzten Lösungsmittels durchgeführt werden. Üblicherweise werden Temperaturen zwischen 20 und 160°C, vorzugsweise 60 bis 120°C, angewendet. Geeignete organische Lösungsmittel sind aromatische Kohlenwasserstoffe - wie Benzol, Toluol, Ethylbenzol, Dichlorbenzol, o-, m-, p-Xylol, Methylnaphthalin-, aliphatische oder cycloaliphatische Kohlenwasserstoffe - wie Ligroin, Heptan, Cyclohexan - Ether - wie Diethylether, Tetrahydrofuran -, Amide - wie N,N-Dimethylformamid, N,N-Dimethylacetamid - N-Methylpyrrolidon oder Mischungen dieser Lösungsmittel.

Bevorzugt erfolgt die Herstellung der Verbindungen I, $R^3$ = CHO, in einer niederen Alkancarbonsäure, insbesondere in Essigsäure bei Temperaturen zwischen 60°C und 120°C, insbesondere bei 70°C bis 100°C.

Die Herstellung der Amine I, $R^3 = CH_2NR^4R^5$, in der $R^5$ eine $C_{1-6}$-Alkylgruppe bedeutet, wird bevorzugt in aromatischen Kohlenwasserstoffen als Lösungsmittel, insbesondere in Toluol, in Gegenwart von 100 bis 150 Molprozent einer organischen Sulfonsäure, insbesondere Toluolsulfonsäure, bei Temperaturen von 90°C bis zur Rückflußtemperatur, insbesondere bei Rückflußtemperatur des verwendeten Lösungsmittels durchgeführt.

Die Herstellung der Amine der Formel I, $R^3 = CH_2NR^4R^5$, in der $R^5$ ein Wasserstoffatom oder die Formylgruppe bedeutet, wird bevorzugt durch die Umsetzung der Verbindungen der Formel III, in der $R^3$ den Rest

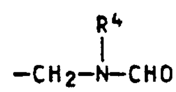

bedeutet in einem Lösungsmittel, wie einer niederen Alkancarbonsäure, insbesondere Essigsäure bei 70°C bis 100°C oder in einem aromatischen Kohlenwasserstoff, wie Toluol, in Gegenwart von 0,1 bis 10 Molprozent einer organischen Sulfonsäure, wie Toluolsulfonsäure bei 90°C bis Ruckflußtemperatur durchgeführt. Die Formylgruppe kann anschließend - falls gewünscht - durch alkalische Hydrolyse mit NaOH abgespalten werden.

Verbindungen der Formel I, in denen $R^3$ den Rest -$CH_2NR^4R^5$, worin $R^5$ nicht den Formylrest bedeutet, konnen durch reduktive Aminierung der Verbindungen der Formel I, $R^3$ = Formyl mit Aminen der Formel $R^4R^5NH$, worin $R^5$ nicht die Formylgruppe bedeutet, erhalten werden. Die reduktive Aminierung kann in einem geeigneten Lösungsmittel beispielsweise mit einem Platin-, Palladium-, Nickel- oder Kobaltkatalysator und Wasserstoff oder mit metallorganischen Hydriden, wie Natriumborhydrid, Natriumcyanborhydrid oder nach Leuckart-Wallach mit Ameisensäure durchgeführt werden. Verbindungen der Formel I, in der $R^3$ die Gruppe $CH_2NHR^4$ bedeutet, können anschließend zu den Verbindungen I, $R^3$ =

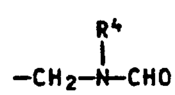

formyliert werden.

Verbindungen der Formel I, in denen $R^3$ den Hydroxymethylrest darstellt, können durch Reduktion der entsprechenden Formylverbindungen hergestellt werden. Diese Reduktion kann beispielsweise mit

Wasserstoff und einem Platin-, Palladium-, Nickel- oder Kobaltkatalysator oder mit metallorganischen Hydriden, wie Natriumborhydrid, Lithiumaluminiumhydrid durchgeführt werden.

Die erfindungsgemäßen Verbindungen besitzen wertvolle thrombocytenaggregationshemmende und cardiotone Eigenschaften. Außerdem senken sie den Blutdruck und hemmen die Magensäuresekretion und übertreffen darin die Vergleichsverbindung [p-(Pyrrol-1-yl)-phenyl]-4,5-dihydropyridazin-3-on (EP-A-75 436) deutlich.

Die neuen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsform ab. In der Regel beträgt die tägliche Wirkstoffdosis zwischen etwa 0,1 bis 10, vorzugsweise 0,5 bis 5 mg bei parenteraler und 1 bis 100, vorzugsweise 5 bis 50 mg bei oraler Anwendung pro Patient und Tag.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Lösungen oder Suppositorien. Diese werden in üblicher Weise hergestellt. Die Wirkstoffe können dabei mit den gebräuchlichen galenischen Hilfsmitteln, wie Tablettenbindern, Füllstoffe, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart 1978). Die so erhaltenen Zubereitungen enthalten den Wirkstoff normalerweise in einer Menge von 0,1 bis 99 Gew.-%.

Die Erfindung wird nachstehend anhand von Beispielen erläutert.

**Beispiel 1A**

Zu 40,6 g (200 mMol) 6-(4-Aminophenyl)-4,5-dihydro-5-methyl-pyridazinon in 400 ml Eisessig wurde eine Lösung von 33,6 g (210 mMol) 2,5-Dimethoxytetrahydrofuran-3-carbaldehyd in 200 ml Eisessig bei 80°C zugetropft. Nach 2 h wurde die Reaktionsmischung in gesättigte $NaHCO_3$-Lösung eingegossen und mit $CH_2Cl_2$ extrahiert. Der Extakt wurde mit Wasser und gesättigter NaCl-Lösung gewaschen und über $Na_2SO_2$ getrocknet. Nach Abziehen des Lösungsmittels am Rotationsverdampfer wurde aus Dimethylformamid (DMF)/Wasser umkristallisiert. Man erhielt 44,2 g 6-[p-(3-Formyl-pyrrol-1-yl)-phenyl]-4,5-dihydro-5-methylpyridazin-3-on, Fp. 191-192°C.

Analog wurden durch Umsetzung der entsprechenden 6-(4-Aminophenyl)-pyridazinone (II) mit 2,5-Dimethoxytetrahydrofuran-3-carbaldehyd die Verbindungen der Formel I, $R^3$ = CHO erhalten bzw. lassen sich erstellen:

| Beispiel | $R^6$ | $R^1$ | $R^2$ | A | B | Fp [°C] |
|---|---|---|---|---|---|---|
| 1B | H | H | H | H | H | 192 194 a) |
| 1C | H | $-(CH_2)-$ | | H | H | 241 - 242 |
| 1D | H | $-(CH_2)_2-$ | | H | H | 186 - 188 |
| 1E | H | H | $CH_3$ | H | H | 192 - 194 |
| 1F | H | H | H | gemeinsame Bindung | | 256 - 258 a) |
| 1G | H | H | $CH_3$ | gemeinsame Bindung | | 228 - 230 b) |
| 1H | H | $CH_3$ | H | gemeinsame Bindung | | > 260 |
| 1I | H | $CH_2OH$ | H | H | H | |
| 1K | gemeinsame Bindung | | H | H | H | |
| 1L | $-CH_2$ | | H | H | H | |
| 1M | $-(CH_2)_2-$ | | H | H | H | 225 227 c) |
| 1N | $-(CH_2)_3$ | | H | H | H | |

a) Der bei der Reaktion entstehende polymere Niederschlag wurde mit Aceton ausgekocht, der Rückstand der Acetonphase wurde mit dem Extrakt der wäßrigen Lösung vereinigt.
b) Umkristallisation aus Methanol.
c) Umkristallisation aus Aceton/Petrolether.

**Beispiel 2A**

4,2 g (15 mMol) 6-[p-(3-Formylpyrrol-1-yl)-phenyl]-4,5-dihydro-5-methyl-pyridazin-3-on (Beispiel 1A) wurden in 50 ml Ethanol und 20 ml Dimethylformamid (DMF) gelöst und mit 1,15 g (30 mMol) Natriumborhydrid versetzt. Nach 6 h Rühren bei 60°C (Umsatzkontrolle mit DC, $SiO_2$, $CH_2Cl_2/CH_3OH$ 9 : 1) wurde mit 50 ml Wasser versetzt und mit Essigester extrahiert. Nach Trocknen der Essigesterphase und Abziehen des Lösungsmittels am Rotationsverdampfer erhielt man eine gelbe kristalline Festsubstanz. Nach Umkristallisation aus $DMF/H_2O$

erhielt man 2,5 g 6-[p-(3-Hydroxymethyl-pyrrol-1-yl)phenyl]-4,5-dihydro-5-methylpyridazin-3-on, Fp. 184 - 186°C.

Analog wurden durch Umsetzung der Verbindungen der Beispiele 1B bis 1N mit Natriumborhydrid die Verbindungen der Beispiele 2B bis 2N I, $R^3 = CH_2OH$ erhalten bzw. lassen sich erhalten:

| Beispiel | $R^6$ | $R^1$ | $R^2$ | A | B | Fp [°C] |
|---|---|---|---|---|---|---|
| 2B | H | H | H | H | H | > 260 a) |
| 2C | H | $-(CH_2)-$ | | H | H | 214 216 b) |
| 2D | H | $-(CH_2)_2-$ | | H | H | amorph |
| 2E | H | H | $CH_3$ | H | H | 198 - 200 c) |
| 2F | H | H | H | gemeinsame Bindung | | 243 - 244 |
| 2G | H | H | $CH_3$ | gemeinsame Bindung | | 253 - 255 |
| 2H | H | $CH_3$ | H | gemeinsame Bindung | | ab 225 (Zers.) c) |
| 2I | H | $CH_2OH$ | H | H | H | |
| 2K | gemeinsame Bindung | | H | H | H | |
| 2L | | $-CH_2-$ | H | H | H | |
| 2M | | $-(CH2)_2-$ | H | H | H | 223 - 224 |
| 2N | | $-(CH2)_3-$ | H | H | H | |

a) Die Substanz fiel nach dem Verdünnen der Reaktionsmischung mit Wasser in reiner Form aus.
b) Nach der Umkristallisation aus DMF/$H_2O$ wurde noch aus Methanol umkristallisiert.
c) Umkristallisation aus Methanol.

**Beispiel 3A**

4,2 g (15 mMol) 6-[p-(3-Formylpyrrol-1-yl)-phenyl]-4,5-dihydro-5-methylpyridazin-3-on (Verbindung aus Beispiel 1A) wurden in 30 ml Methanol mit 3,36 40 %-iger wäßriger Dimethylaminlösung (30 mMol), 1,8 g (30 mMol) Essigsäure und 0,75 g (12 mMol) Natriumcyanborhydrid 5 h bei Raumtemperatur gerührt (Umsatzkontrolle mit Dünnschichtchromatographie, $SiO_2$, Essigester/Essigsäure/Wasser 5 : 2 : 2). Man versetzte mit 50 ml $H_2O$, stellte mit 2 N NaOH alkalisch, extrahierte mit Essigester, wusch die organische Phase mit Wasser und trocknete mit $Na_2SO_4$. Nach Reinigung des Extraktes über eine Kieselgelsäule mit Methylenchlorid/Methanol (4 : 1) wurde der kristalline Rückstand der produkthältigen Fraktionen aus Essigester/-Petrolether umkristallisiert. Man erhielt 1,4 g 6-[p-(3-Dimethylaminomethylpyrrol-1-yl)-phenyl]-4,5-dihydro-5-methylpyridazin-3-on, Fp. 167 bis 168°C.

Analog Beispiel 3A wurden durch Umsetzung der Verbindung des Beispiels 1A mit den Aminen $R^4R^5NH$ die Verbindungen der Beispiele 3B bis 3M erhalten.
I, $R^1 = CH_3$, $R^2 = R^6 = A = B = H$,
$R^3 = -CH_2-NR^4R^5$

| Beispiel | $R^4$ | $R^5$ | Fp [°C] |
|---|---|---|---|
| 3B | $-(CH_2)_5-$ | | 76 - 77 |
| 3C | $C_6H_5CH_2$ | $CH_3$ | 237 - 238 |
| 3D | $n-C_4H_9$ | $n-C_4H_9$ | 88 - 90 a) |
| 3E | $i-C_3H_7$ | H | 92 - 94 |
| 3F | $n-C_6H_{13}$ | H | ab 105 (Zers.) a) |
| 3G | $C_6H_5-CH_2-$ | H | 135 - 137 b) |
| 3H | $C_6H_5-CH(CH_3)-$ | H | ab 125 (Zers.) a) |
| 3I | $-(CH_2)_4-$ | | 92 - 93 a) |
| 3K | $-(CH_2)_6-$ | | 98 - 100 a) |
| 3L | $-(CH_2)_7-$ | | 136 - 137 c) |
| 3M | $CH_3$ | H | 72 - 75 d) |

a) Nach der Extraktion wurde das Rohprodukt in Methanol mit 1 Äquivalent Weinsäure versetzt und nach Abziehen des Methanols aus Isopropanol umkristallisiert.
b) Umkristallisation aus DMF/Ether und aus Isopropanol.
c) Umkristallisation aus Essigester.
d) Tartrat aus Dimethylformamid/Ether umkristallisiert.

**Beispiele 4 bis 15**

Analog Beispiel 3A wurden durch Umsetzung der Verbindungen der Beispiele 1B-1N mit Aminen $R^4R^5NH$ die folgenden Verbindungen der Beispiele 4 bis 15 erhalten werden bzw. lassen sich herstellen:

**Beispiel 4A bis 4M**

I, $R^1 = R^2 = R^6 = A = B = H$, $R^3 = CH_2NR^4R^5$

| Beispiel | $R^4$ | $R^5$ | Fp [°C] |
|---|---|---|---|
| 4A | $CH_3$ | $CH_3$ | amorph a) |
| 4B | $-(CH_2)_5-$ | | |
| 4C | $C_6H_5-CH_2$ | $CH_3$ | |
| 4D | $n-C_4H_9$ | $n-C_4H_9$ | amorph a) |
| 4E | $i-C_3H_7$ | H | |
| 4F | $n-C_6H_{13}$ | H | |
| 4G | $C_6H_5CH_2$ | H | |
| 4H | $C_6H_5(CH_2)_3-$ | H | |
| 4I | $-(CH_2)_4-$ | | |
| 4K | $-(CH_2)_6-$ | | |
| 4L | $-(CH_2)_7-$ | | |
| 4M | $CH_3$ | H | |

a) Substanz wurde als Tartrat aus Isopropanol umkristallisiert.

**Beispiel 5A - 5M**

I, $R^1 + R^2 = -CH_2-$, $R^6 = A = B = H$, $R^3 = -CH_2NR^4R^5$

| Beispiel | $R^4$ | $R^5$ | Fp [°C] |
|---|---|---|---|
| 5A | $CH_3$ | $CH_3$ | |
| 5B | $-(CH_2)_5-$ | | amorph a) |
| 5C | $C_6H_5-CH_2$ | $CH_3$ | |
| 5D | $n-C_4H_9$ | $n-C_4H_9$ | |
| 5E | $i-C_3H_7$ | H | |
| 5F | $n-C_6H_{13}$ | H | |
| 5G | $C_6H_5CH_2$ | H | |
| 5H | $C_6H_5(CH_2)_2-$ | H | |
| 5I | $-(CH_2)_4-$ | | |
| 5K | $-(CH_2)_6-$ | | |
| 5L | $-(CH_2)_7-$ | | |
| 5M | $CH_3$ | H | |

a) Substanz wurde als Tartrat aus Isopropanol umkristallisiert.

**Beispiele 6A bis 6M**

I, $R^1 + R^2 = -(CH_2)_2-$, $R^6 = A = B = H$, $R^3 = -CH_2NR^4R^5$

| Beispiel | $R^4$ | $R^5$ | Fp[°C] |
|---|---|---|---|
| 6A | $CH_3$ | $CH_3$ | |
| 6B | $-(CH_2)_5-$ | | |
| 6C | $C_6H_5-CH_2$ | $CH_3$ | amorph a) |
| 6D | $n-C_4H_9$ | $n-C_4H_9$ | |
| 6E | $i-C_3H_7$ | H | |
| 6F | $n-C_6H_{13}$ | H | |
| 6G | $C_6H_5CH_2$ | H | |
| 6H | $C_6H_5(CH_2)_2-$ | H | |
| 6I | $-(CH_2)_4-$ | | |
| 6K | $-(CH_2)_6-$ | | |
| 6L | $-(CH_2)_7-$ | | |
| 6M | $CH_3$ | H | |

a) Substanz wurde als Tartrat aus Isopropanol umkristallisiert.

**Beispiele 7A bis 7M**

I, $R^1 = H$, $R^2 = CH_3$, $R^6 = A = B = H$, $R^3 = -CH_2NR^4R^5$

| Beispiel | $R^4$ | $R^5$ | Fp [°C] |
|---|---|---|---|
| 7A | $CH_3$ | $CH_3$ | |
| 7B | $-(CH_2)_5-$ | | |
| 7C | $C_6H_5-CH_2$ | $CH_3$ | |
| 7D | $n-C_4H_9$ | $n-C_4H_9$ | |
| 7E | $i-C_3H_7$ | H | 220 - 222 a) |
| 7F | $n-C_6H_{13}$ | H | |
| 7G | $C_6H_5CH_2$ | H | |
| 7H | $C_6H_5(CH_2)_2-$ | H | |
| 7I | $-(CH_2)_4-$ | | |
| 7K | $-(CH_2)_6-$ | | |
| 7L | $-(CH_2)_7-$ | | |
| 7M | $CH_3$ | H | |

a) Semitartrat aus Isopropanol umkristallisiert.

**Beispiele 8A bis 8M**

I, $R^1 = R^2 = R^6 = H$, A + B = gemeinsame Bindung, $R^3 = -CH_2NR^4R^5$

| Beiseiel | $R^4$ | $R^5$ | Fp [°C] |
|---|---|---|---|
| 8A | $CH_3$ | $CH_3$ | |
| 8B | $-(CH_2)_5-$ | | 220 - 222 a) |
| 8C | $C_6H_5-CH_2$ | $CH_3$ | |
| 8D | $n-C_4H_9$ | $n-C_4H_9$ | |
| 8E | $i-C_3H_7$ | H | |
| 8F | $n-C_6H_{13}$ | H | |
| 8G | $C_6H_5CH_2$ | H | |
| 8H | $C_6H_5(CH_2)_2-$ | H | |
| 8I | $-(CH_2)_4-$ | | |
| 8K | $-(CH_2)_6-$ | | |
| 8L | $-(CH_2)_7-$ | | |
| 8M | $CH_3$ | H | |

a) Umkristallisation aus Methanol.

**Beispiel 9A bis 9M**

I, $R^1 = R^6 = H$, $R^2 = CH_3$, A + B = gemeinsame Bindung, $R^3 = -CH_2NR^4R^5$

| Beispiel | $R^4$ | $R^5$ | Fp [°C] |
|---|---|---|---|
| 9A | $CH_3$ | $CH_3$ | |
| 9B | $-(CH_2)_5-$ | | |
| 9C | $C_6H_5-CH_2$ | $CH_3$ | |
| 9D | $n-C_4H_9$ | $n-C_4H_9$ | |
| 9E | $i-C_3H_7$ | H | |
| 9F | $n-C_6H_{13}$ | H | amorph a) |
| 9G | $C_6H_5CH_2$ | H | |
| 9H | $C_6H_5(CH_2)_2-$ | H | |
| 9I | $-(CH_2)_4-$ | | |
| 9K | $-(CH_2)_6-$ | | |
| 9L | $-(CH_2)_7-$ | | |
| 9M | $CH_3$ | H | |

a) Tartrat aus Methanol/Ether umkristallisiert.

**Beispiele 10A bis 10M**

I, $R^1 = CH_3$, $R^2 = R^6 = H$, A + B = gemeinsame Bindung, $R^3 = -CH_2NR^4R^5$

| Beispiel | $R^4$ | $R^5$ | Fp [°C] |
|---|---|---|---|
| 10A | $CH_3$ | $CH_3$ | |
| 10B | $-(CH_2)_5-$ | | |
| 10C | $C_6H_5-CH_2$ | $CH_3$ | |
| 10D | $n-C_4H_9$ | $n-C_4H_9$ | |
| 10E | $i-C_3H_7$ | H | |
| 10F | $n-C_6H_{13}$ | H | |
| 10G | $C_6H_5CH_2$ | H | |
| 10H | $C_6H_5(CH_2)_2-$ | H | |
| 10I | $-(CH_2)_4-$ | | 183 - 185 a) |
| 10K | $-(CH_2)_6-$ | | |
| 10L | $-(CH_2)_7-$ | | |
| 10M | $CH_3$ | H | |

a) Umkristallisation aus Essigester.

**Beispiele 11A bis 11M**

I, $R^1 = CH_2OH$, $R^2 = R^6 = A = B = H$, $R^3 = CH_2NR^4R^5$

| Beispiel | $R^4$ | $R^5$ |
|---|---|---|
| 11A | $CH_3$ | $CH_3$ |
| 11B | $-(CH_2)_5-$ | |
| 11C | $C_6H_5-CH_2$ | $CH_3$ |
| 11D | $n-C_4H_9$ | $n-C_4H_9$ |
| 11E | $i-C_3H_7$ | H |
| 11F | $n-C_6H_{13}$ | H |
| 11G | $C_6H_5CH_2$ | H |
| 11H | $C_6H_5(CH_2)_2-$ | H |
| 11I | $-(CH_2)_4-$ | |
| 11K | $-(CH_2)_6-$ | |
| 11L | $-(CH_2)_7-$ | |
| 11M | $CH_3$ | H |

**Beispiele 12A bis 12M**

I, $R^1 + R^6$ = gemeinsame Bindung, $R^2 = A = B = H$, $R^3 = CH_2NR^4R^5$

| Beispiel | $R^4$ | $R^5$ |
|---|---|---|
| 12A | $CH_3$ | $CH_3$ |
| 12B | $-(CH_2)_5-$ | |
| 12C | $C_6H_5-CH_2$ | $CH_3$ |
| 12D | $n-C_4H_9$ | $n-C_4H_9$ |
| 12E | $i-C_3H_7$ | H |
| 12F | $n-C_6H_{13}$ | H |
| 12G | $C_6H_5CH_2$ | H |
| 12H | $C_6H_5(CH_2)_2-$ | H |
| 12I | $-(CH_2)_4-$ | |
| 12K | $-(CH_2)_6-$ | |
| 12L | $-(CH_2)_7-$ | |
| 12M | $CH_3$ | H |

**Beispiele 13A bis 13M**

I, $R^1 + R^5 = -CH_2-$, $R^2 = A = B = H$, $R^3 = CH_2NR^4R^5$

| Beispiel | $R^4$ | $R^5$ |
|---|---|---|
| 13A | $CH_3$ | $CH_3$ |
| 13B | $-(CH_2)_5-$ | |
| 13C | $C_6H_5-CH_2$ | $CH_3$ |
| 13D | $n-C_4H_9$ | $n-C_4H_9$ |
| 13E | $i-C_3H_7$ | H |
| 13F | $n-C_6H_{13}$ | H |
| 13G | $C_6H_5CH_2$ | H |
| 13H | $C_6H_5(CH_2)_2-$ | H |
| 13I | $-(CH_2)_4-$ | |
| 13K | $-(CH_2)_6-$ | |
| 13L | $-(CH_2)_7-$ | |
| 13M | $CH_3$ | H |

**Beispiele 14A bis 14M**

I, $R^1 + R^6 = -CH_2CH_2-$, $R^2 = A = B = H$, $R^3 = CH_2NR^4R^5$

| Beispiel | $R^4$ | $R^5$ |
|---|---|---|
| 14A | $CH_3$ | $CH_3$ |
| 14B | $-(CH_2)_5-$ | |
| 14C | $C_6H_5-CH_2$ | $CH_3$ |
| 14D | $n-C_4H_9$ | $n-C_4H_9$ |
| 14E | $i-C_3H_7$ | H |
| 14F | $n-C_6H_{13}$ | H |
| 14G | $C_6H_5CH_2$ | H |
| 14H | $C_6H_5(CH_2)_2-$ | H |
| 14I | $-(CH_2)_4-$ | |
| 14K | $-(CH_2)_6-$ | |
| 14L | $-(CH_2)_7-$ | |
| 14M | $CH_3$ | H |

**Beispiele 15A bis 15M**

I, $R^1 + R^6 = -(CH_2)_3-$, $R^2 = A = B = H$, $R^3 = CH_2NR^4R^5$

| Beispiel | $R^4$ | $R^5$ |
|---|---|---|
| 15A | $CH_3$ | $CH_3$ |
| 15B | $-(CH_2)_5-$ | |
| 15C | $C_6H_5-CH_2$ | $CH_3$ |
| 15D | $n-C_4H_9$ | $n-C_4H_9$ |
| 15E | $i-C_3H_7$ | H |
| 15F | $n-C_6H_{13}$ | H |
| 15G | $C_6H_5CH_2$ | H |
| 15H | $C_6H_5(CH_2)_2-$ | H |
| 15I | $-(CH_2)_4-$ | |
| 15K | $-(CH_2)_6-$ | |
| 15L | $-(CH_2)_7-$ | |
| 15M | $CH_3$ | H |

**Beispiel 16**

1,0 g (5 mMol) 6-(4-Aminophenyl)-4,5-dihydro-5-methyl-pyridazin-3-on und 1,0 g (5 mMol) 2,5-Dimethoxy-3(N-formyl-N-methylaminomethyl)-tetrahydro-furan wurden in 10 ml Essigsäure 2 h bei 80°C gerührt. Man goß in 250 ml gesättigte $KHCO_3$-Lösung, extrahierte mit $CH_2Cl_2$. Nach Trocknen des Extraktes über $Na_2SO_4$ und Abziehen des Lösungsmittels wurde der Rückstand aus Aceton/Petrolether umkristallisiert. Man erhielt 1,8 g 6-[4-(3-N-Formyl-N-methylaminomethylpyrrol-1-yl)-phenyl]-4,5-dihydro-5-methyl-pyridazin-3-on, Fp. 148 - 150°C.

**Beispiel 17**

5 g (15 mMol) 6-[4-(3-N-Formyl-N-methylaminomethylpyrrol-1-yl)-phenyl]-4,5-dihydro-5-methyl-pyridazin-3-on (Beispiel 16) wurden in 200 ml Methanol mit 75 ml 1N NaOH drei Tage bei 50°C gerührt. Nach Abziehen des Lösungsmittels wurde mit wäßriger Essigsäure versetzt und mit Essigester extrahiert. Die saure Wasserphase wurde nach Alkalisieren mit 2 N NaOH mit Essigester extrahiert. Diese organische phase wurde über $Na_2SO_4$ getrocknet. Nach Abziehen des Lösungsmittels wurde der Rückstand mit 1 Äquivalent Weinsäure in Ethanol versetzt, vom Lösungsmittel befreit und aus Dimethylformamid/Ether umkristallisiert. Man erhielt 5,4 g 6-[4-(3-N-Methyl-aminomethylpyrrol-1-yl-phenyl]-4,5-dihydro-5-methyl-pyridazin-3-on, Fp. 73 bis 75°C.

**Beispiel 18A**

10 mMol 6-[4-Aminophenyl)-4,5-dihydro-5-methyl-pyridazinon wurden in Toluol unter Zusatz von wenig Dimethylformamid mit 13 mMol Toluolsulfonsäure und 12 mMol 2,5-Dimethoxy-3-dimethylaminomethyl-tetrahydrofuran im Wasserauskreiser unter Rückfluß gekocht. Nach dem dünnschichtchromatografisch bestimmten Ende der Reaktion wurde das Lösungsmittel zum großen Teil abgezogen, der Rückstand in Dimethylformamid aufgenommen und in, gesättigte $NaHCO_3$-Lösung gegossen. Nach Extraktion der wäßrigen Phase mit $CH_2Cl_2$ wurde der organische Extrakt über $Na_2SO_4$ getrocknet und am Rotationsverdampfer eingeengt. Das Rohprodukt wurde mittels Säulenchromatographie ($SiO_2$, $CH_2Cl_2/CH_3OH$) gereinigt. Man erhielt 6-[4-(3-Dimethylaminomethylpyrrol-1-yl)-phenyl]-4,5-dihydro-5-methylpyridazin-3-on, das mit der in Beispiel 3A synthetisierten Substanz identisch war.

Analog Beispiel 18A können durch Umsetzung der entsprechenden 6-(4-Amino-phenyl)-pyridazinone mit 2,5-Dimethoxy-3-dimethylaminomethyl-tetrahydro-furan bzw. mit 2,5-Dimethoxy-3-piperidinomethyl-tetrahydrofuran die Verbindungen der Beispiele 18B-K erhalten werden.

| Beispiel | $R^6$ | $R^1$ | $R^2$ | A | B | $R^4$ | $R^5$ |
|---|---|---|---|---|---|---|---|
| 18B | H | $CH_3$ | H | H | H | $-(CH_2)_5-$ | |
| 18C | H | H | H | H | H | $CH_3$ | $CH_3$ |
| 18D | H | H | H | H | H | $-(CH_2)_5-$ | |
| 18E | H | $-CH_2-$ | | H | H | $CH_3$ | $CH_3$ |
| 18F | H | $-CH_2-$ | | H | H | $-(CH_2)_5-$ | |
| 18G | H | H | H | gemeinsame Bindung | | $CH_3$ | $CH_3$ |
| 18H | H | H | H | gemeinsame Bindung | | $-(CH_2)_5-$ | |
| 18I | $-(CH_2)_2-$ | | H | H | H | $CH_3$ | $CH_3$ |
| 18K | $-(CH_2)_2-$ | | H | H | H | $-(CH_2)_5-$ | |

**Beispiel 19**

Analog Beispiel 18A wurde durch Umsetzung von 1 g (5 mMol) 6-(4-Aminophenyl)-4,5-dihydro-5-methyl-pyridazin-3-on mit 1,0 g (5 mMol) 2,5-Dimethoxy-3-(N-formyl-N-methyl-aminomethyl)-tetrahydrofuran und 0,1 g (0,5 mMol) Toluolsulfonsäure in einer Mischung aus 100 ml Toluol und 10 ml DMF 6-[4-(3-N-Formyl-N-methyaminomethylpyrrol-1-yl)-phenyl]-4,5-dihydro-5-methylpyridazin-3-on erhalten, das mit der Verbindung aus Beispiel 16 identisch ist.

**Beispiel 20A**

Eine Lösung von 3,24 g (10 mMol) 6-[4-(3-N-Isopropylaminomethyl-pyrrol-1-yl)-phenyl]-4,5-dihydro-5-methylpyridazin-3-on, 0,05 g (1 mMol) Natriummethylat und 10 g (167 mMol) Ameisensäuremethylester in 10 ml Dimethylformamid wurden über Nacht bei Raumtemperatur gerührt. Man goß in 100 ml Wasser und extrahierte mit Methylenchlorid. Nach Trocknen über Natriumsulfat und Abziehen des Lösungsmittels wurde der Rückstand säulenchromatographisch (Kieselgel, $CH_2Cl_2/CH_3OH$ 4 : 1) gereinigt und aus Methanol umkristallisiert. Man erhielt 1,25 g 6-[4-(3-N-Formyl-N-isopropylaminomethylpyrrol-1-yl)-phenyl]-4,5-dihydro-5-methylpyridazin-3-on, Fp. 180 - 181°C.

Analog Beispiel 20A können durch Umsetzung der Verbindungen I, $R^3 = CH_2NHR^4$ mit Ameisensäuremethylester die Verbindungen der Beispiele 20B - 20AD erhalten werden.

$$I,\ R^3 = CH_2\text{-}\overset{\overset{\large R^4}{|}}{N}\text{-}CHO$$

| Beispiel | $R^6$ | $R^1$ | $R^2$ | A | B | $R^4$ |
|---|---|---|---|---|---|---|
| 20B | H | $CH_3$ | H | H | H | n-$C_6H_{13}$ |
| 20C | H | $CH_3$ | H | H | H | $C_6H_5$-$CH_2$- |
| 20D | H | $CH_3$ | H | H | H | $C_6H_5$-$CH(CH_3)$- |
| 20E | H | H | H | H | H | $CH_3$- |
| 20F | H | H | H | H | H | i-$C_3H_7$ |
| 20G | H | H | H | H | H | n-$C_6H_{13}$- |
| 20I | H | H | H | H | H | $C_6H_5$-$CH_2$- |
| 20K | H | -$CH_2$- | | H | H | $CH_3$- |
| 20L | H | -$CH_2$- | | H | H | i-$C_3H_7$- |
| 20M | H | -$CH_2$- | | H | H | n-$C_6H_{13}$- |
| 20N | H | -$CH_2$- | | H | H | $C_6H_5CH_2$- |
| 20O | H | -$(CH_2)_2$- | | H | H | $CH_3$- |
| 20P | H | -$(CH_2)_2$- | | H | H | i-$C_3H_7$- |
| 20Q | H | -$(CH_2)_2$- | | H | H | n-$C_6H_{13}$- |
| 20R | H | -$(CH_2)_2$- | | H | H | $C_6H_5$-$CH_2$- |
| 20S | H | H | $CH_3$ | H | H | $CH_3$- |
| 20T | H | H | $CH_3$ | H | H | i-$C_3H_7$- |
| 20U | H | H | $CH_3$ | H | H | n-$C_6H_{13}$- |
| 20V | H | H | $CH_3$ | H | H | $C_6H_5CH_2$- |
| 20W | H | H | H | gemeinsame Bindung | | $CH_3$- |
| 20X | H | H | H | gemeinsame Bindung | | i-$C_3H_7$- |
| 20Y | H | H | H | gemeinsame Bindung | | n-$C_6H_{13}$- |
| 20Z | H | H | H | gemeinsame Bindung | | $C_6H_5CH_2$- |
| 20AA | -$(CH_2)_2$- | | H | H | H | $CH_3$- |
| 20AB | -$(CH_2)_2$- | | H | H | H | i-$C_3H_7$- |
| 20AC | -$(CH_2)_2$- | | H | H | H | n-$C_6H_{13}$- |
| 20AD | -$(CH_2)_2$- | | H | H | H | $C_6H_5CH_2$- |

## Patentansprüche

1. (Pyrrol-1-yl)-phenyl-dihydropyridazinone der Formel I

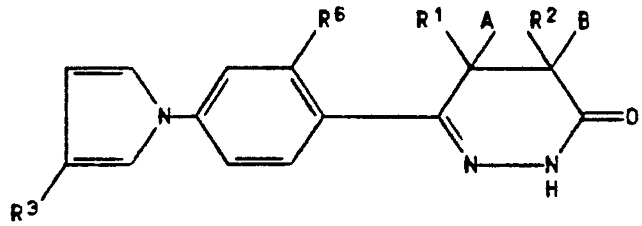

I,

worin

$R^1$ ein Wasserstoffatom, eine Methylgruppe oder eine Hydroxymethylgruppe,

$R^2$ ein Wasserstoffatom oder eine Methylgruppe, oder $R^1$ und $R^2$ zusammen einen Methylen- oder Ethylenrest,

A und B Wasserstoffatome oder - falls $R^1$ und $R^2$ keine Alkylengruppe darstellen - auch eine gemeinsame Bindung sind,

$R^3$ eine Formyl- oder Hydroxymethylgruppe oder eine Gruppe $CH_2$-$NR^4R^5$ bedeutet, worin $R^4$ eine $C_{1-6}$-Alkylgruppe oder eine Phenyl-$C_{1-3}$-alkylengruppe und $R^5$ ein Wasserstoffatom oder eine $C_{1-6}$-Alkylgruppe oder die Formylgruppe sind, oder $R^4$ und $R^5$ zusammen einen $C_{4-7}$-Alkylenrest bedeuten, und

$R^6$ ein Wasserstoffatom bedeutet oder zusammen mit $R^1$ den Rest -$(CH_2)_m$- bedeutet. worin m für 0, 1, 2 oder 3 steht, sowie gegebenenfalls deren Säureadditionssalze.

2. Verfahren zur Herstellung der Verbindungen gemäß Anspruch 1, dadurch gekennzeichnet, daß man p-Aminophenyl-dihydropyridazinone der Formel II

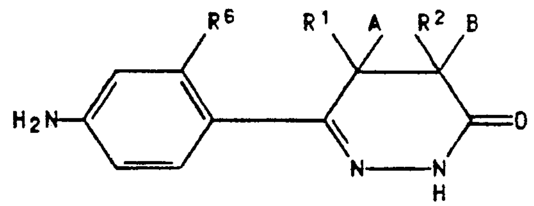

II,

worin $R^1$, $R^2$, $R^6$, A und B die angegebene Bedeutung besitzen mit Tetrahydrofuranen der Formel III

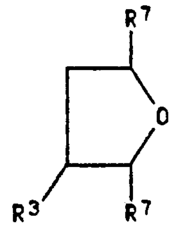

III,

worin $R^3$ die angegebene Bedeutung hat und $R^7$ $C_{1-4}$-Alkoxy-, $C_{1-4}$-Acyloxyreste, Chlor- oder Bromatome bedeuten, umsetzt, und die so erhaltenen Verbindungen gegebenenfalls - falls $R^3$ = Formyl -reduziert (→ I, $R^3$ = $CH_2OH$) oder durch reduktive Aminierung in die Amine I ($R^3$ = $CH_2NR^4R^5$) überführt.

3. Verbindungen der Formel I gemäß Anspruch 1 zur Verwendung bei der Bekämpfung von Krankheiten.

**Revendications**

1. (Pyrrol-1-yl)-phényl-dihydropyridazinones de la formule I

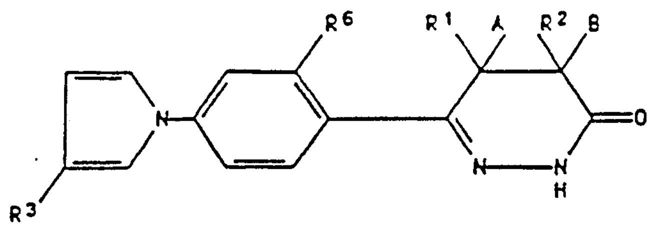

I

dans laquelle

$R^1$ représente un atome d'hydrogène, un radical méthyle ou un radical hydroxyméthyle,

$R^2$ représente un atome d'hydrogène ou un radical méthyle, ou bien $R^1$ et $R^2$ représentent ensemble un radical méthylène ou éthylène,

A et B représentent des atomes d'hydrogène ou également - dans le cas où $R^1$ et $R^2$ ne représentent ni l'un ni l'autre un radical alkylène - une liaison commune,

$R^3$ représente un radical formyle ou hydroxyméthyle, ou un radical $CH_2$-$NR^4R^5$ où $R^4$ représente un radical alkyle en $C_1$-$C_6$, ou un radical phénylalkylène ($C_1$-$C_3$) et $R^5$ représente un atome d'hydrogène ou un radical alkyle en $C_1$-$C_6$, ou bien le radical formyle, ou bien $R^4$ et $R^5$ représentent ensemble un radical alkylène en $C_4$-$C_7$, et

$R^6$ représente un atome d'hydrogène, ou forme ensemble avec $R^1$ le radical $-(CH_2)_m$ - où m est égal à 0, 1, 2 ou 3, comme aussi leurs sels d'addition d'acides éventuels.

2. Procédé de préparation des composés suivant la revendication 1, caractérisé en ce que l'on fait réagir des p-aminophényl-dihydropyridazinones de la formule II

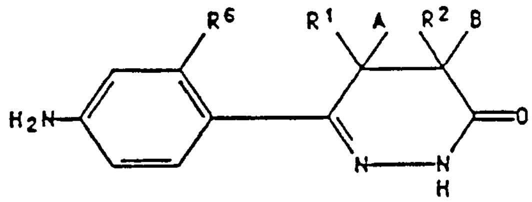

II

dans laquelle $R^1$, $R^2$, $R^6$, A et B possèdent les significations qui leur ont été précédemment attribuées, sur des tétrahydrofurannes de la formule III

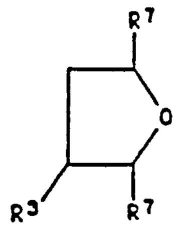

III

dans laquelle $R^3$ possède les significations qui lui ont été précédemment attribuées et $R^7$ représente un radical alcoxy en $C_1$-$C_4$, acyloxy en $C_1$-$C_4$, des atomes de chlore ou àe brome, et on réduit éventuellement les composés ainsi obtenus - dans le cas où $R^3$ = formyle - (→ I, $R^3$ = $CH_2OH$), ou bien on les transforme en les amines I ($R^3$ = $CH_2NR^4R^5$) par amination réductrice.

3. Composés de la formule I suivant la revendication 1, en vue de leur utilisation pour lutter contre des malades.

## Claims

1. A pyrrol-1-ylphenyldihydropyridazinone of the formula I

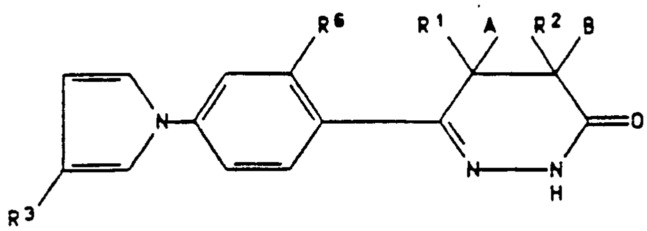

(I)

where $R^1$ is hydrogen, methyl or hydroxymethyl, $R^2$ is hydrogen or methyl, or $R^1$ and $R^2$ together form a methylene or ethylene radical, A and B are each hydrogen or, where $R^1$ and $R^2$ do not form an alkylene group, they furthermore may be a common bond, $R^3$ is formyl or hydroxymethyl or $CH_2$-$NR^4R^5$ where $R^4$ is $C_1$-$C_6$-alkyl or phenyl-$C_1$-$C_3$-alkylene and $R^5$ is hydrogen, $C_1$-$C_6$-alkyl or formyl or $R^4$ and $R^5$ together form a $C_4$-$C_7$-alkylene radical, and $R^6$ is hydrogen or, together with $R^1$, forms a radical $-(CH_2)_m-$, where m is 0, 1, 2 or 3, and, if appropriate, its addition salts with acids.

2. A process for the preparation of a compound as claimed in claim 1, wherein a p-aminophenyldihydropyridaznone of the formula II

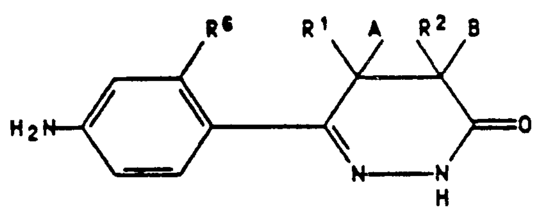

(II)

where $R^1$, $R^2$, $R^6$, A and B have the stated meanings, is reacted with a tetrahydrofuran of the formula III

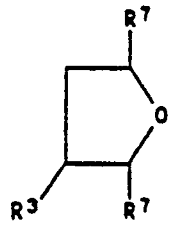

(III)

where $R^3$ has the stated meanings and $R^7$ is $C_1$-$C_4$-alkoxy, $C_1$-$C_4$-acyloxy, chlorine or bromine, and, if desired, the compound thus obtained is reduced if $R^3$ is formyl (→ I, $R^3$ = $CH_2OH$), or is converted to an amine I ($R^3$ = $CH_2NR^4R^5$) by reductive amination.

3. A compound of the formula I as claimed in claim 1 for use in the treatment of disorders.